# FASCICULE DE BREVET EUROPEEN

(11) **EP 0 820 766 B1**
(45) Date de publication et mention de la délivrance du brevet: **09.04.2003**
(21) Numéro de dépôt: 97401632.1
(22) Date de dépôt: 08.07.1997
(51) Int. Cl.: A61K 7/48, A61K 7/06

(54) **Nouvelles compositions topiques comprenant de très faibles doses de mélatonine ou ses dérivés et leur utilisation en cosmétique**
Neue topische Zusammensetzungen, die Melatonin oder seine Analogen in sehr niedrigen Dosen enthalten, und ihre Verwendung in der Kosmetik
New topical compositions containing very low doses of melatonin or derivatives thereof and their use in cosmetics

(30) Priorité: 25.07.1996 FR 9609387
(43) Date de publication de la demande: 28.01.1998
(73) Titulaire: L'OREAL, 75008 Paris (FR)
(72) Inventeur: Tachon, Pierre, 92160 Antony (FR); Nadaud, Jean-François, 92140 Clamart (FR); Pruche, Francis, 75018 Paris (FR); Breton, Lionel, 78000 Versailles (FR); Gerst, Catherine, 92600 Asnieres (FR)
(74) Mandataire: Tezier Herman, Béatrice

(56) Documents cités:
- WO-A-86/05093
- WO-A-87/00432
- WO-A-93/07870

## Description

La présente invention a pour objet de nouvelles compositions dermo-cosmétiques comprenant de la mélatonine ou au moins un de ses analogues à des doses appropriées et présentant une activité antioxydante améliorée, en particulier pour améliorer ou maintenir l'apparence de la peau ou du scalp.

Le rôle de l'oxydation cellulaire sur le vieillissement cutané, qu'il soit intrinsèque ou extrinsèque, notamment photo-induit, est connu. Ce vieillissement cutané se traduit par différents signes cliniques notamment l'apparition de ridules et de rides profondes, en augmentation avec l'âge. Par ailleurs, l'apparence de la peau ou du scalp se détériore. Le teint de la peau est généralement modifié et il peut exister sur certaines zones de la peau des irritations diffuses et parfois des télangiectasies. Un autre signe clinique du vieillissement est l'aspect sec et rêche de la peau qui est dû essentiellement à une desquamation plus importante. On constate enfin une perte de fermeté et de tonicité de la peau qui, comme pour les rides et les ridules, s'explique du moins en partie par une atrophie dermique et épidermique ainsi qu'un aplatissement de la formation. On constate donc que les signes cliniques du vieillissement cutané résultent essentiellement d'un dysfonctionnement des principaux mécanismes biologiques intervenant au niveau de la peau.

Prévenir ou traiter le vieillissement cutané, qu'il soit intrinsèque ou extrinsèque, et les signes cliniques décrits ci-dessus, revient à maintenir ou améliorer l'apparence de la peau ou du scalp.

Différents antioxydants susceptibles de prévenir ou traiter le vieillissement cutané sont décrits dans l'état de la technique, par exemple la mélatonine (EP 214 254).

La mélatonine, ou N-acétyl-5-méthoxytryptamine, surtout connue pour son activité sur le rythme circadien régulant la production d'hormones, est également décrite pour son activité antioxydante (Reiter R J, *Verhandung der Deutschen Zoologischen Gesellschaft*, 87 (2), 1994, 195-204; Reiter R J & al., *Neuroendocrinoll Letter*, 15 (1-3), 1993, 103-113; Reiter R J & al., *J. Pineal Res.,* 18 (1), 1995, 1-11), en particulier son activité antiradicalaire (Reiter RJ & al., *Brazilian Journal of Medical and Biological Research,* 26 (22), 1993, 1141-1155). La plupart des études des propriétés antioxydantes de la mélatonine portent sur les phénomènes d'oxydation liés au vieillissement du cerveau (Poeggeler B & al., *J. Pineal Res.,* 14 (4), 1993, 151-168; Cagnoli C M & al., *J. Pineal Res.,* 18 (4), 1995, 222-226; Melchiorri D & al., *FASEB J.,* 9 (12), 1995, 1205-1210; Sewerynek E & al., *Neuroscience Letters*, 195 (3), 1995, 203-205.

La mélatonine a également été décrite pour son utilisation en dermo-cosmétique pour améliorer l'apparence de la peau (JP 61 221 104; WO 86/05093), ou pour protéger la peau contre l'effet d'une irradiation aux rayonnements U.V. (EP 0 438 856; E. Bangha & al., Dermatology 191, [2], 176, 1995). Il est préconisé d'employer la mélatonine à des concentrations comprises entre 10⁻⁴ et 10% en poids par rapport au poids total de la composition.

Ainsi, la demande de brevet WO 86/05093, décrit une composition cosmétique comprenant de la mélatonine pour augmenter la sensibilité de la peau aux oestrogènes, en particulier pour le traitement de l'acné ou la prévention de la chute des cheveux. Les quantités de mélatonine préconisées dans cette demande sont comprises d'une manière générale entre 10⁻⁴ et plus de 1% en poids par rapport au poids total de la composition. Les deux seuls exemples spécifiques de compositions, lotion pour le traitement de l'acné et solution pour prévenir la chute des cheveux, comprennent 10% et 0,1 % en poids de mélatonine, respectivement.

De même la demande de brevet WO 87/00432 décrit l'utilisation de la mélatonine pour traiter le psoriasis. Les quantités de mélatonine préconisées dans cette demande sont comprises d'une manière générale entre 10⁻⁴ et plus de 1% en poids par rapport au poids total de la composition.

De même, la demande de brevet EP 438 856, concernant des compositions pour la protection de la peau contre les effets des irradiations aux rayonnements U.V., préconise des quantités de mélatonine comprises entre 1 et 10% en poids pour des compostions topiques, le seul exemple de ce type de composition comprenant 10% de mélatonine.

La demande de brevet JP 61 221 104 décrit pour sa part un lait de toilette comprenant 10⁻⁴% de mélatonine pour atténuer la rugosité de la peau. Comme pour les deux publications précédentes, l'action antioxydante de la mélatonine n'est pas mentionnée pour les effets recherchés.

L'article de Bangha & al. (Dermatology 191, [2], 176, 1995) décrit un gel contenant de la mélatonine capable de réduire l'érythème induit par les U.V., probablement compte-tenu de son activité antiradicaux libres. Les concentrations essayées sont de 0,05, 0,1 et 0,5 %, les auteurs préconisant une concentration de 0,5 %.

La demanderesse a maintenant démontré que de manière surprenante, la mélatonine était inefficace à des doses correspondant aux concentrations décrites dans la littérature, mais était efficace comme antioxydant à des doses inférieures, proches des doses physiologiques.

La présente invention concerne donc l'utilisation de la mélatonine ou au moins un de ses analogues dans des compositions topiques à des concentrations inférieures à celles préconisées dans l'état de la technique de manière à obtenir une activité antioxydante identique, voire améliorée.

En effet, on a pu mettre en évidence sur des kératinocytes humains normaux en culture, que la mélatonine ne présentait pas d'activité antioxydante à une concentration de 10⁻⁵ M, mais était active pour des concentrations inférieures ou égales à 10⁻⁷M.

De même, on a pu mettre en évidence une activité antioxydante de type hormonal au niveau cellulaire, en particulier une activité inductrice de l'expression des mRNAs de la glutathion péroxydase (GSH), enzyme clé de la défense antioxydante de la peau, à des doses physiologiques allant de 10⁻⁹ jusqu'à 10⁻¹⁵ M.

La présente invention concerne donc une composition topique comprenant de la mélatonine ou au moins un de ses analogues et un support cosmétiquement ou pharmaceutiquement acceptable, dans laquelle la quantité de mélatonine ou ses analogues est une quantité appropriée pour permettre après application topique, le passage dans la peau de doses physiologiques de mélatonine ou ses analogues, comprises entre 10⁻¹⁵ et 10⁻⁷ M.

La présente invention concerne également une composition comprenant de la mélatonine ou au moins un de ses analogues et un support support cosmétiquement ou pharmaceutiquement acceptable, dans laquelle la quantité de mélatonine ou ses analogues étant strictement inférieure à 10⁻⁴ % en poids par rapport au poids total de la composition.

De manière préférentielle, la quantité de mélatonine ou au moins un de ses analogues dans la composition selon l'invention, est inférieure ou égale à 0,5.10⁻⁴ % en poids, plus préférentiellement inférieure ou égale à 10⁻⁶ % en poids, avantageusement comprise entre 10⁻¹² % et 10⁻⁵ % en poids par rapport au poids total de la composition.

Parmi les analogues de la mélatonine, on peut notamment citer ses dérivés, tels que la 5-méthoxytryptamine, 5-méthoxytryptophane, 5-méthoxytryptophol, l'acide 5-méthoxyindole-3-acétique et la 6-hydroxymélatonine. On peut également citer des agonistes mélatoninergiques, tels que ceux décrits dans les demandes de brevets WO 95/17405, EP 0 447 285, EP 0 527 687, EP 0 530 087 et EP 0 591 057. Ces composés sont d'origine naturelle ou synthétique.

La composition topique dans laquelle se trouve la mélatonine ou au moins un de ses analogues, est une composition cosmétique ou dermatologique et peut se présenter sous toutes les formes galéniques à usage topique normalement utilisées et le support physiologiquement acceptable peut être constitué par tout support usuel pour une composition cosmétique ou dermatologique. La composition peut avoir la forme notamment de solution aqueuse ou suspension huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Les quantités des différents constituants des compositions sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosasée, le psoriasis, les lichens, les prurits sévères.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique ou dermatologique peut contenir également des adjuvants habituels dans le domaine cosmétique ou dermatologique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans ces domaines, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose® 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

Dans la mesure où ils n'interfèrent pas avec l'activité de la mélatonine, les compositions selon l'invention peuvent contenir d'autres actifs destinés notamment à la prévention et/ou au traitement des affections cutanées.

Les compositions selon l'invention sont particulièrement appropriées pour prévenir ou traiter un stress oxydatif de la peau et/ou de ses annexes, en particulier lié aux U.V., au vieillissement, à l'inflammation, à l'alopécie.

D'autres caractéristiques de l'invention apparaîtront à la lumière des exemples ci-après. Dans les compositions, les proportions indiquées sont des pourcentages en poids.

### EXEMPLES

### Exemple 1: Etude sur kératinocytes humains normaux en culture

Des kératinocytes humains normaux ont été incubés pendant une heure en présence de mélatonine à différentes concentrations. Les cellules ont été soumises à un stress radicalaire provoqué par des rayonnements U.V. A (10 J/cm³). Les hydroperoxydes sont détectés dans les cellules par une sonde fluorescente (CM-H2DCF-DA, commercialisée par Molecular Probe).

Les résultats sont résumés dans le Tableau I ci-dessous, comparés à des témoins cultivés en l'absence de mélatonine, avec ou sans irradiation, et à une référence (cellules cultivées en présence d'une association vitamine C 0,5 mg/ml et GSH 0,5 mg/ml).

| Concentration en Mélatonine (M) | Unités de fluorescence par mesure | % de protection |
|---|---|---|
| 10⁻⁵ M | 1123 ± 50 | - |
| 10⁻⁷ M | 863 ± 152 | 34,0 |
| 10⁻⁹ M | 802 ± 123 | 42,5 |
| témoin irradié | 1135 ± 90 | - |
| témoin non irradié | 350 ± 7 | - |
| référence (Vit C + GSH) | 932 ± 10 | 26,0 |

Ces résultats démontrent une activité antioxydante nulle à des doses de mélatonine de 10⁻⁵ M, c'est à dire correspondant aux compositions décrites dans l'état de la technique, et l'apparition d'une activité antioxydante importante à des concentrations inférieures.

### Exemple 2 : Etude de la stimulation de la production de GSH-Px dans les kératinocytes interfolliculaires

### Stimulation de l'activité de la GSH-Px

Des kératinocytes humains normaux ont été incubés pendant des temps croissants (1h30, 3h et 6h) en présence de mélatonine à des concentrations variant de 10⁻⁶ à 10⁻¹⁷ M. Le contrôle correspond à des kératinocytes incubés en l'absence de mélatonine.

Après incubation, les kératinocytes sont repris dans un tampon d'homogénéisation. Les kératinocytes sont soumis à trois cycles de congélation/décongélation puis l'extrait est centrifugé et le dosage de la GSH-Px est effectué sur le surnageant par la technique au TBH décrite par D.E. Paglia & W.N. Valentine (*J. Lab. Clin. Med.,* 70, 1967, 158-169).

On observe une stimulation de l'activité de la GSH-Px dès 1h30 de traitement à la mélatonine avec un pic à 10⁻¹⁴ M, concentration à laquelle on observe 60 % d'augmentation par rapport au témoin non traité.

### Stimulation de l'expression de la GSH-Px

Des kératinocytes humains normaux ont été incubés pendant des temps croissants (1h30, 3h et 6h) en présence de mélatonine à des concentrations variant de 10⁻⁶ à 10⁻¹⁷ M. Le contrôle correspond à des kératinocytes incubés en l'absence de mélatonine.

Le niveau d'expression de la GSH-Px est mesuré après RT-PCR des ARNm de la GSH-Px. Le niveau d'expression de la GSH-Px pour chaque condition expérimentale est rapporté à celui de l'actine qui représente le contrôle interne.

On observe une augmentation du niveau d'expression de la GSH-Px après 1h30 d'incubation avec la mélatonine, avec un pic à 10⁻¹⁵ M (60 % d'augmentation). Cette augmentation se retrouve après 3h et 6h d'incubation, avec un pourcentage inférieur à 6h (40 %).

Ces résultats démontrent une stimulation de la GSH-Px par la mélatonine à des concentrations physiologiques (jusqu'à 10⁻¹⁷ M), de type hormonal avec une dose optimale à 10⁻¹⁴ et 10⁻¹⁵ M.

### Exemple 3 : Etude sur fibroblastes humains normaux en culture

Des fibroblastes ont été obtenus par la technique de culture d'explants. Les cellules ont été ensemencées dans des plaques de culture de 6 puits. Le milieu de culture de fibroblastes (MCF) est constitué par le milieu DMEM/M199 (3/1 v/v), additionné de glutamine (2 mM), de pénicilline (50 Ul/ml), de streptomycine (50 µg/ml), de bicarbonate de sodium (0,2 % p/v) et de sérum de veau foetal (10 % v/v).

Les fibroblastes sont incubés dans le milieu MCF à 37 °C dans une atmosphère humide contenant 5 % de CO₂, jusqu'à confluence des monocouches. Ils sont incubés en présence de fer-NTA pour provoquer la production des hyperoxydes qui sont responsables d'une atteinte radicalaire importante.

Les produits de référence sont l'acétate de vitamine E à 1mg/ml (référence 1) et la déféroxamine à 6,5 mg/ml (référence 2).

La mélatonine et les produits de référence sont directement solubilisés dans le tampon contenant la sonde fluorescente à 5 µM (CM-H2DCF-DA).

Les fibroblastes sont incubés avec la mélatonine ou les produits de référence pendant un heure précédent l'addition de fer-NTA, puis pendant l'incubation en présence de fer-NTA.

Des fibroblastes témoins ont été cultivés en absence de mélatonine ou de produits de référence, en présence ou en absence de fer-NTA.

Après incubation avec le fer-NTA, les fibroblastes sont lavés par addition de milieu, puis sont lysés par action des ultra-sons. Les lysats cellulaires sont transférés dans des plaques de 96 puits. En présence d'hydroperoxydes, la sonde CM-H2DCF-DA devient fluorescente (A.S. Keston & R. Brandt, Anal. Biochem., 11, 1965, 1-5). La fluorescence de la sonde a été mesurée par fluorimétrie à l'aide d'un analyseur à plaques (excitation : 355 mm, émission : 460 mm).

Les résultats sont résumés dans le Tableau II ci-dessous, comparés à des témoins cultivés en l'absence de mélatonine, avec ou sans fer-NTA, et à deux références.

| Concentration en Mélatonine (M) | Unités de fluorescence par mesure | % de protection |
|---|---|---|
| 10⁻⁷ M | 6539 ± 1413 | 86,9 |
| 10⁻⁸ M | 4940 ± 1045 | 100 |
| 10⁻⁹M | 5655 ± 997 | 100 |
| 10⁻¹⁰ M | 5043 ± 705 | 100 |
| 10⁻¹¹M | 4817 ± 1141 | 100 |
| témoin sans Fe-NTA | 5721 ± 607 | - |
| témoin avec Fe-NTA | 11958 ± 1573 | - |
| référence 1 | 4197 ± 443 | 100 |
| référence 2 | 5688 ± 264 | 100 |

Les résultats ci-dessus confirment l'activité antioxydante de la mélatonine à des concentrations physiologiques, les pourcentages de protection obtenus étant équivalents à ceux observés pour les deux références employées à des concentrations beaucoup plus importantes (de l'ordre de 10⁻² M).

### Exemple 4: Crème de soin du visage (émulsion huile dans eau)

| | | |
|---|---|---|
| 6-hydroxy-mélatonine | | 0,5 10⁻⁶ |
| Stéarate de glycérol | | 2,00 |
| Polysorbate 60 (Tween 60 vendu par la société ICI) | | 1,00 |
| Acide stéarique | | 1,40 |
| Triéthanolamine | | 0,70 |
| Carbomer | | 0,40 |
| Fraction liquide du beurre de karité | | 12,00 |
| Perhydrosqualène | | 12,00 |
| Antioxydant | | 0,05 |
| Parfum | | 0,5 |
| Conservateur | | 0,30 |
| Eau | qsp | 100 % |

### Exemple 5: Lotion de soin

| | | |
|---|---|---|
| mélatonine | | 1 10⁻⁶ |
| Glycérol | | 2,00 |
| Méthyl Paraben | | 0,15 |
| Parfum | qsp | |
| Eau déminéralisée Stérile | qsp | 100 % |

## Revendications

1. Composition topique comprenant de la mélatonine ou ses analogues et un support cosmétiquement ou pharmaceutiquement acceptable, **caractérisée en ce que** la quantité de mélatonine ou au moins un de ses analogues est strictement inférieure à 10⁻⁴ % en poids par rapport au poids total de la composition.

2. Composition selon la revendication 1, **caractérisée en ce que** la quantité de mélatonine ou au moins un de ses analogues est inférieure ou égale à 0,5 10⁻⁴ % en poids.

3. Composition selon la revendication 2, caractérisée en que la quantité de mélatonine ou au moins un de ses analogues est comprise entre 10⁻¹² % et 10⁻⁵ % en poids.

4. Composition selon l'une des revendications 1 à 3, **caractérisée en ce que** les analogues sont choisis parmi la 5-méthoxytryptamine, la 5-méthoxytryptophane, le 5-méthoxytryptophol, l'acide 5-méthoxyindole-3-acétique et la 6-hydroxymélatonine.

5. Composition selon l'une des revendications 1 à 4, **caractérisée en ce qu'**il s'agit d'une composition cosmétique ou dermatologique.

6. Composition selon l'une des revendications 1 à 5, **caractérisée en ce qu'**elle se présente sous la forme de solution aqueuse ou suspension huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique.

## Patentansprüche

1. Kosmetische Zusammensetzung, die Melatonin oder mindestens einer Verbindung, die Melatonin analog ist, und einen kosmetisch oder pharmazeutisch akzeptablen Träger enthält, **dadurch gekennzeichnet, daß** der Mengenanteil von Melatonin oder mindestens einer Verbindung, die Melatonin analog ist, unter 10⁻⁴ Gew.-%, bezogen auf das Gesamtgewicht der Zusammensetzung, liegt.

2. Zusammensetzung nach Anspruch 1, **dadurch gekennzeichnet, daß** der Mengenanteil des Melatonin oder mindestens einer Verbindung, die Melatonin analog ist, 0,5 ·10⁻⁴ Gew.-% beträgt oder darunter liegt.

3. Zusammensetzung nach Anspruch 2, **dadurch gekennzeichnet, daß** der Mengenanteil des Melatonin oder mindestens einer Verbindung, die Melatonin analog ist, im Bereich von 10⁻¹² bis 10⁻⁵ Gew.-% liegt.

4. Zusammensetzung nach einem der Ansprüche 1 bis 3, **dadurch gekennzeichnet, daß** die Analogen von Melatonin unter 5-Methoxytryptamin, 5-Methoxytryptophan, 5-Methoxytryptophol, 5-Methoxyindol-3-essigsäure oder 6-Hydroxymelatonin ausgewählt sind.

5. Zusammensetzung nach einem der Ansprüche 1 bis 4, **dadurch gekennzeichnet, daß** es sich um eine kosmetische oder dermatologische Zusammensetzung handelt.

6. Zusammensetzung nach einem der Ansprüche 1 bis 5, **dadurch gekennzeichnet, daß** sie als wäßrige Lösung, ölige Suspension, Dispersion vom Typ Lotion oder Serum, Emulsion flüssiger oder halbflüssiger Konsistenz vom Typ Milch, die durch Dispersion einer Fettphase in einer wäßrigen Phase (O/W) oder umgekehrt (W/O) hergestellt wird, Suspension oder Emulsion von weicher Konsistenz vom Typ Creme oder wäßriges oder wasserfreies Gel oder auch in Form von Mikrokapseln, Mikropartikeln oder Vesikeldispersionen vom ionischen und/oder nichtionischen Typ vorliegt.

## Claims

1. Topical composition comprising melatonin or analogues thereof and a cosmetically or pharmaceutically acceptable support, **characterized in that** the amount of melatonin or of at least one analogue thereof is strictly less than 10⁻⁴% by weight relative to the total weight of the composition.

2. Composition according to Claim 1, **characterized in that** the amount of melatonin or of at least one analogue thereof is less than or equal to 0.5 × 10⁻⁴% by weight.

3. Composition according to Claim 2, **characterized in that** the amount of melatonin or of at least one analogue thereof is between 10⁻¹²% and 10⁻⁵% by weight.

4. Composition according to one of Claims 1 to 3, **characterized in that** the analogues are chosen from 5-methoxytryptamine, 5-methoxytryptophan, 5-methoxytryptophol, 5-methoxyindole-3-acetic acid and 6-hydroxymelatonin.

5. Composition according to one of Claims 1 to 4, **characterized in that** it is a cosmetic or dermatological composition.

6. Composition according to one of Claims 1 to 5, **characterized in that** it is in the form of an aqueous solution or oily suspension or dispersion of the lotion or serum type, emulsions of liquid or semi-liquid consistency of the milk type, obtained by dispersion of a fatty phase in an aqueous phase (O/W) or conversely (W/O), or suspensions or emulsions of soft consistency of the cream or aqueous or anhydrous gel type, or alternatively microcapsules or microparticles, or vesicule dispersions of ionic and/or nonionic type.
